# EUROPEAN PATENT APPLICATION

(11) **EP 3 040 026 A1**
(43) Date of publication of application: **06.07.2016**
(21) Application number: 14841148.1
(22) Date of filing: 21.08.2014
(51) Int. Cl.: A61B 6/00, A61B 6/03, A61B 5/00, A61B 5/055

(54) **BIOLOGICAL TISSUE DIAGNOSIS APPARATUS AND METHOD**

(30) Priority: 28.08.2013 KR 20130102415; 16.07.2014 KR 20140089525
(71) Applicant: Vieworks Co., Ltd., Anyang-si, Gyeonggi-do 431-060 (KR)
(72) Inventor: LEE, Su Gil, Seongnam-si Gyeonggi-do 463-956 (KR); KANG, Yu Jung, Paju-si Gyeonggi-do 413-965 (KR); CHOI, Chul Hee, Daejeon 305-338 (KR)
(74) Representative: Zacco Sweden AB
(86) International application number: PCT/KR2014/007771
(87) International publication number: WO 2015/030424

(57) **Abstract**

The present invention relates to a biological tissue diagnosis apparatus, which comprises: at least one lighting control unit for irradiating a contrast agent excitation energy on at least one tissue of interest within a living body in which an imaging contrast agent has been injected; at least one inspection unit for imaging an energy radiated from the at least one tissue of interest; and a determination unit for determining, on the basis of the image data imaged by the at least one inspection unit, whether a tube or perfusion of the at least one tissue of interest is abnormal.

## Description

### [Technical Field]

The present invention relates to a biological tissue diagnostic apparatus and method, and more particularly, to a biological tissue diagnostic apparatus and method which analyze image data obtained by detecting energy emitted from tissues of interest *in-vivo* with a contrast agent administered thereto, thereby accurately diagnosing whether vessels or perfusion of the tissues of interest is abnormal.

### [Background Art]

As a method of quantitatively measuring blood perfusion of tissues, SPECT, PET, MRI angiography, and the like are used in clinics. However, since such methods require expensive equipment and troublesome processes and are costly, they are only used in measurement of blood perfusion of tissues directly relating to life, such as the heart and the brain.

Accordingly, although diseases such as hypertension, diabetes, and adult diseases are increasing in an aging society and have a huge effect on quality of life, there is no technology capable of functionally and quantitatively measuring blood flow of biological tissues.

As a method for measuring peripheral arterial disease of lower limbs, CT-angiography estimates perfusion of a tissue based on information on an anatomical vascular structure obtained through an image and thus cannot provide accurate information on blood flow. In addition, an ankle-brachial index (ABI) technique is developed to determine abnormality of the leg artery based on the ratio of blood pressures of arms and legs and is applied to a patient suspected to have peripheral arterial disease. However, when the artery has been calcified or the collateral artery has developed a lot, this technique is likely to make a mistake of diagnosing a tissue perfusion rate lower than an actual value <Kashyap VS, 2008; Luetkemeier MJ, 2001>. Further, there is no technique proposing a quantitative blood flow measurement method for research on functions of blood vessels/lymphatic vessel system through animal testing and research on development of treatment drugs for blood vessel/lymphatic vessel diseases.

Typical angiography using indocyanine green (ICG) (ICG angiography) has been proven safe and is clinically used in measurement of angiogenesis of a transplanted skin or a degree of ocular neovascularization of a diabetic patient.

When irradiated with near-infrared light at 730 nm to 790 nm, ICG emits fluorescent light in a longer wavelength range of 800 nm to 860 nm, which can be measured using a camera or spectrometer. Near-infrared light has penetrability and is less scattered and thus has been actively studied as technology for photographing the human body.

### <References>

1. Kashyap VS, Pavkov ML, Bishop PD, Nassoiy SP, Eagleton MJ, et al. (2008) Angiography underestimates peripheral atherosclerosis: lumenography revisited. J Endovasc Ther 15: 117-125.
2. Luetkemeier MJ, Fattor JA (2001) Measurement of Indocyanine Green dye is improved by use of polyethylene glycol to reduce plasma turbidity. Clin Chem 47: 1843-1845.

### [Disclosure]

### [Technical Problem]

It is one aspect of the present invention to provide a biological tissue diagnostic apparatus and method which can continuously photograph tissues of interest *in-vivo,* particularly, peripheral tissues, blood vessels of which are distributed near the skin, thereby allowing easy detecting with near-infrared light, and carotid territories for a certain period of time, thereby accurately diagnosing abnormality of vessels or perfusion of the tissues of interest.

It is another aspect of the present invention to provide a biological tissue diagnostic apparatus and method which can simultaneously or selectively photograph one or more tissues *in-vivo,* specifically both hands and both feet, to diagnose abnormality of blood vessels or lymphatic vessels, thereby performing accurate diagnosis through complex diagnosis while reducing diagnostic time.

### [Technical Solution]

In accordance with one aspect of the present invention, a biological tissue diagnostic apparatus includes: at least one lighting unit irradiating at least one tissue of interest *in-vivo* with a contrast agent administered thereto with contrast agent excitation energy; at least one inspection unit detecting energy emitted from the at least one tissue of interest; and a determination unit determining abnormality of vessels or perfusion of the at least one tissue of interest based on image data detected by the at least one inspection unit.

The determination unit may include: a pattern processor patterning a change of the image data over time; a characteristic value calculator calculating at least one characteristic value based on patterned data; and a diagnostic unit diagnosing abnormality of vessels or perfusion of the at least one tissue of interest based on the at least one characteristic value or combinations thereof.

The tissue of interest may include a plurality of tissues of interest, and the diagnostic unit may further diagnose an abnormal tissue of interest through comparison of characteristic values of the tissues of interest.

In diagnosis of an abnormal tissue of interest, the determination unit may diagnose a tissue of interest corresponding to a characteristic value departing from a preset normal distribution range of the characteristic values of the tissues of interest as an abnormal tissue of interest.

When characteristic values for each tissue of interest are acquired from normal groups or abnormal groups of vessels or perfusion, a combination value of one or more characteristic values included in the normal groups or the abnormal groups may allow the groups to be classified with a sensitivity and specificity of 80% or higher.

The at least one characteristic value may be obtained from a change of a specific physical value for a first time period in the tissue of interest obtained from the image data, and the at least one characteristic value obtained for the first time period may include at least one selected from among a period of time from a time point that the physical value is initially detected to a time point that the physical value reaches a maximum value; a slope with which the physical value increases or decreases for a specific period of time; a period of time for which the physical value is maintained at a reference value or more; a period of time from the time point that the physical value is initially detected to a time point before the physical value increases with a slope greater than or equal to a reference slope; and a period of time from a time point that the physical value starts to decrease below the reference value to a time point that the physical value is last detected.

The contrast agent may be an indocyanine green (ICG) pigment.

The inspection unit may include: a filter member transmitting energy in the near-infrared region among energy emitted from the contrast agent; and a detecting member detecting energy in the near-infrared region having passed through the filter member.

The inspection unit may include a plurality of inspection units to independently inspect one or more biological tissues, and the detecting member included in each of the inspection units may be controlled in operation time by a controller.

Operation time of the detecting member may be within a period of time from a time point that detecting is started to a time point that emission of near-infrared rays from the contrast agent is no longer detected, and the detecting member may be controlled to perform detecting at preset time intervals.

The determination unit determining abnormality of vessels or perfusion of the at least one tissue of interest may represent determination results as to abnormality of the vessels or perfusion by one or more numerical values or a perfusion map.

The apparatus may further include: a dark chamber blocking external light to increase contrast of an image, wherein the lighting unit may include an illumination member emitting the contrast agent excitation energy, and the dark chamber may accommodate the illumination member and the inspection unit.

In accordance with another aspect of the present invention, a biological tissue diagnostic method includes: irradiating, by at least one lighting unit, at least one tissue of interest *in-vivo* with a contrast agent administered thereto with contrast agent excitation energy; detecting, by at least one inspection unit, energy emitted from the at least one tissue of interest; and determining, by a determination unit, abnormality of vessels or perfusion of the at least one tissue of interest based on image data detected by the at least one inspection unit.

Determining abnormality of vessels or perfusion of the at least one tissue of interest may include: patterning a change of the image data over time; calculating at least one characteristic value based on the patterned data; and diagnosing abnormality of vessels or perfusion of the at least one tissue of interest based on the at least one characteristic value or combinations of the characteristic values.

The tissue of interest may include a plurality of tissues of interest, and determining abnormality of vessels or perfusion of the at least one tissue of interest may further include: diagnosing an abnormal tissue of interest through comparison of the characteristic values of the tissues of interest.

In diagnosing an abnormal tissue of interest, the determination unit may diagnose a tissue of interest corresponding to characteristic values departing from a preset normal distribution range as an abnormal tissue of interest.

The at least one characteristic value may be obtained from a change of a specific physical value for a first time period in the tissue of interest obtained from the image data, and the at least one characteristic value obtained for the first time period may include at least one selected from among a period of time from a time point that the physical value is initially detected to a time point that the physical value reaches a maximum value; a slope with which the physical value increases or decreases for a specific period of time; a period of time for which the physical value is maintained at a reference value or more; a period of time from the time point that the physical value is initially detected to a time point before the physical value increases with a slope greater than or equal to a reference slope; and a period of time from a time point that the physical value starts to decrease below the reference value to a time point that the physical value is last detected.

### [Advantageous Effects]

According to the present invention, it is possible to provide a biological tissue diagnostic apparatus and method which can continuously photograph tissues of interest *in-vivo* for a certain period of time, particularly blood vessels/ lymphatic vessels which are distributed near the skin, such as peripheral tissues and carotid territoriesallowing easy photographing with near-infrared light, , thereby accurately diagnosing whether vessels or perfusion of the tissues of interest is abnormal.

According to the present invention, it is possible to provide a biological tissue diagnostic apparatus and method which can simultaneously or selectively photograph one or more tissues *in-vivo,* specifically both hands and both feet to diagnose abnormality of blood vessels or lymphatic vessels, thereby performing accurate diagnosis through complex diagnosis while reducing diagnostic time.

### [Description of Drawings]

Fig. 1 is a perspective view of a biological tissue diagnostic apparatus according to one embodiment of the present invention.
Fig. 2 is a sectional view of the biological tissue diagnostic apparatus according to the embodiment of the present invention.
Fig. 3 is a block diagram of the biological tissue diagnostic apparatus according to the embodiment of the present invention.
Fig. 4 is a flowchart illustrating a biological tissue diagnostic method according to one embodiment of the present invention.
Fig. 5 and Fig. 6 are schematic views showing inspection of blood flow of a foot using the biological tissue diagnostic apparatus according to the embodiment of the present invention.
Fig. 7 is a graph obtained by patterning a change of fluorescence intensity from a contrast agent over time after administration of the contrast agent to legs of a person with normal blood flow using the biological tissue diagnostic apparatus according to the embodiment of the present invention.
Fig. 8 is a schematic diagram of a method of defining Onset, Tₘₐₓ, Plateau Tₘₐₓ, and Slope among characteristic values by which a pattern is characterized for comparison of patterned data.
Fig. 9 is a graph obtained by patterning blood flow of hands and legs of a person with normal blood flow using the biological tissue diagnostic apparatus according to the embodiment of the present invention.
Fig. 10 is a graph obtained by patterning blood flow of hands and legs of a person with abnormal blood flow using the biological tissue diagnostic apparatus according to the embodiment of the present invention.
Fig. 11 is a reference view for illustrating a method for diagnosing an abnormal tissue of interest *in-vivo* based on graphs obtained by patterning fluorescence intensity in a plurality of tissues of interest.

### [Best Mode]

Hereinafter, embodiments of the present invention will be described in detail with reference to the accompanying drawings. It should be understood that the present invention may be embodied in different ways and is not limited to the following embodiments. In the drawings, portions irrelevant to the description will be omitted for clarity. Like components will be denoted by like reference numerals throughout the specification.

In addition, as used herein, the terms "includes", "comprises", "including" and/or "comprising" specify the presence of stated features, steps, operations, elements, and/or components, but do not preclude the presence or addition of one or more other features, steps, operations, elements, components, and/or groups.

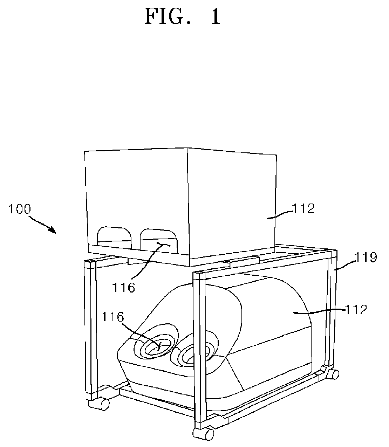

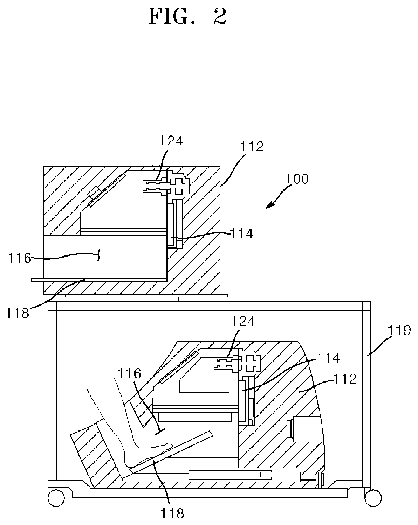

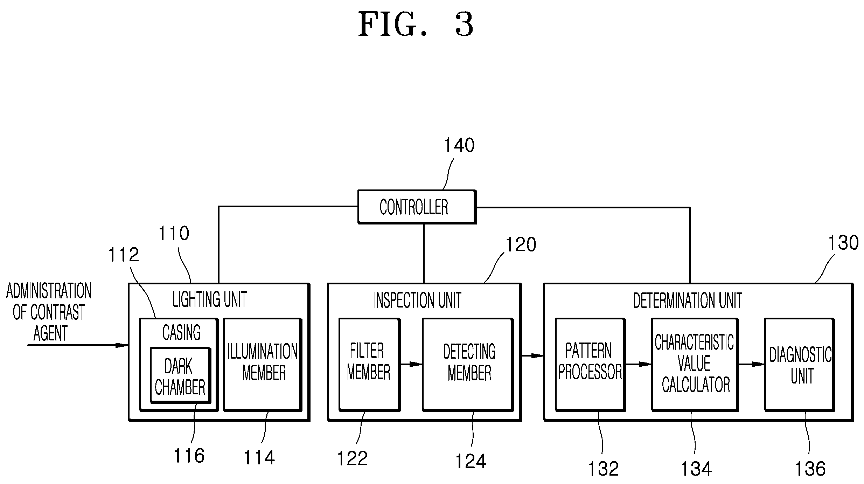

Fig. 1 is a perspective view of a biological tissue diagnostic apparatus according to one embodiment of the present invention; Fig. 2 is a sectional view of the biological tissue diagnostic apparatus according to the embodiment of the present invention; and Fig. 3 is a block diagram of the biological tissue diagnostic apparatus according to the embodiment of the present invention.

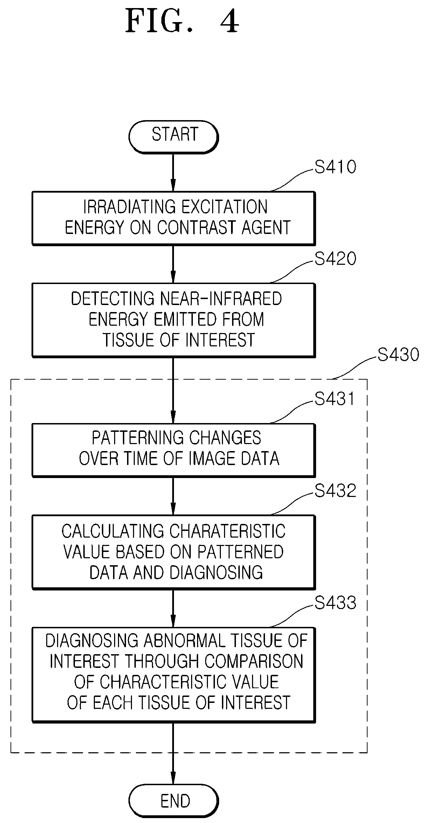

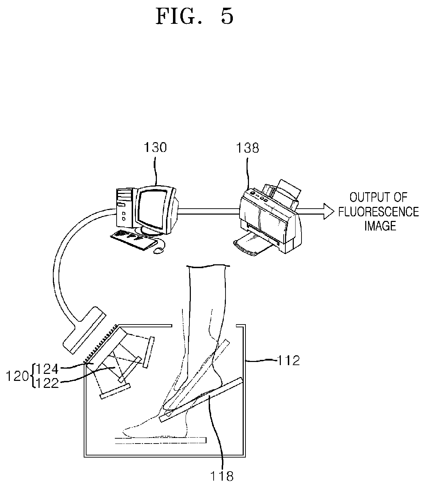

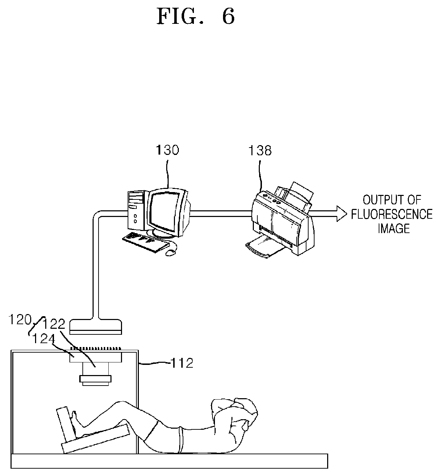

Fig. 4 is a flowchart illustrating a biological tissue diagnostic method according to one embodiment of the present invention; and Fig. 5 and Fig. 6 are schematic views showing inspection of blood flow of legs using the biological tissue diagnostic apparatus according to the embodiment of the present invention.

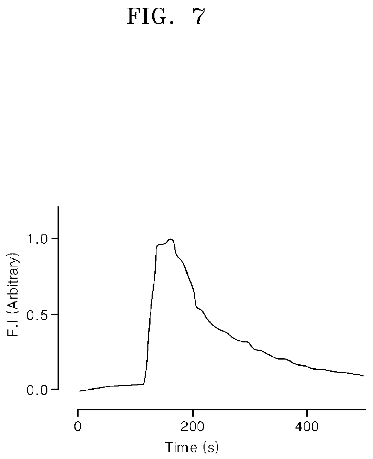

Fig. 7 is a graph obtained by patterning a change of fluorescence intensity from a contrast agent over time after administration of the contrast agent to legs of a person with normal blood flow using the biological tissue diagnostic apparatus according to the embodiment of the present invention.

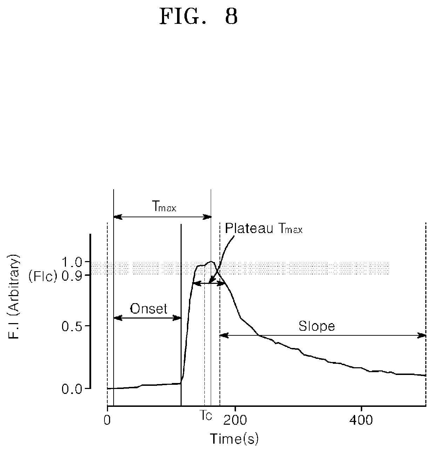

Fig. 8 is a schematic diagram of a method of defining Onset, Tₘₐₓ, Plateau Tₘₐₓ, and Slope among characteristic values by which a pattern is characterized for comparison of patterned data.

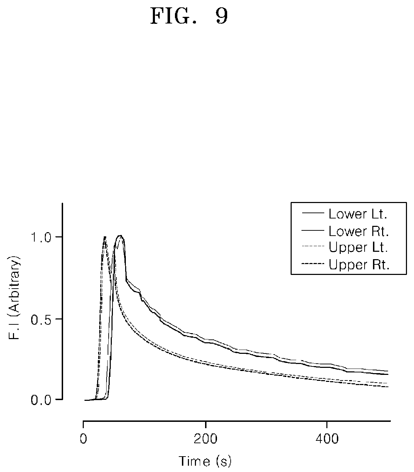

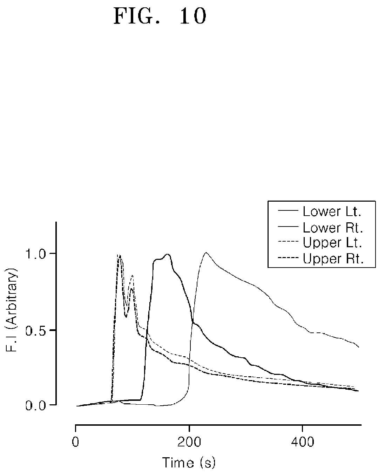

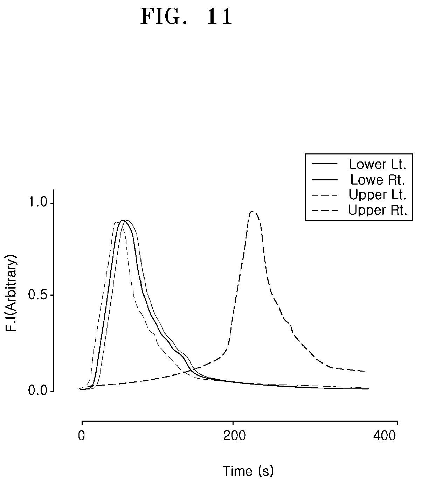

Fig. 9 is a graph obtained by patterning blood flow of hands and legs of a person with normal blood flow using the biological tissue diagnostic apparatus according to the embodiment of the present invention; Fig. 10 is a graph obtained by patterning blood flow of hands and legs of a person with abnormal blood flow using the biological tissue diagnostic apparatus according to the embodiment of the present invention; and Fig. 11 is a reference view for illustrating a method for diagnosing an abnormal tissue of interest *in-vivo* based on graphs obtained by patterning fluorescence intensity of a plurality of tissues of interest.

Referring to Figs. 1 to 3, a biological tissue diagnostic apparatus according to one embodiment of the present invention diagnoses abnormality of vessels or perfusion including blood flow and blood vessels by administering a contrast agent to a biological tissue. The biological tissue diagnostic apparatus according to this embodiment includes: at least one lighting unit 110 irradiating at least one tissue of interest *in-vivo* within a living body with a contrast agent administered thereto with contrast agent excitation energy; at least one inspection unit 120 detecting energy emitted from the contrast agent in the living body; and a determination unit 130 determining abnormality of vessels or perfusion of the at least one tissue of interest based on image data detected by the at least one inspection unit 120.

Here, the determination unit 130 includes: a pattern processor 132 patterning a change of the image data over time; a characteristic value calculator 134 calculating at least one characteristic value based on the patterned data; and a diagnostic unit 136 diagnosing abnormality of vessels or perfusion of the at least one tissue of interest based on the at least one characteristic value or combinations thereof.

As used herein, the term "living body" refers to an animal or human and, for convenience, is shown as being a part of the human body.

In addition, the term "tissue of interest (biological tissue)" may refer to, for example, microvessels of fingertips and toe tips.

Thus, the biological tissue diagnostic apparatus 100 according to this embodiment may examine or diagnose microvessels of fingers of both hands at the same time or may examine or diagnose microvessels of fingers of one hand. In addition, the biological tissue diagnostic apparatus 100 according to this embodiment may examine or diagnose microvessels of toes of both feet at the same time or may examine or diagnose microvessels of toes of one foot.

Further, the biological tissue diagnostic apparatus 100 according to this embodiment may examine or diagnose microvessels of both fingers and toes at the same time or may examine or diagnose microvessels of any one of fingers and toes.

In other words, the biological tissue diagnostic apparatus 100 according to this embodiment may simultaneously or separately examine and/or diagnose perfusion (blood flow, lymphatic flow) or vessels (blood vessels, lymphatic vessels) of a plurality of tissues of interest.

The lighting unit 110 serves to irradiate tissues of interest *in-vivo* with a contrast agent administered thereto. Here, the contrast agent (molecular detecting contrast agent) is a near-infrared fluorescent contrast agent, and is preferably a polymethine pigment, which is a fluorescent pigment, particularly a near-infrared pigment. Particularly, the contrast agent may be an indocyanine green (ICG) pigment.

The contrast agent is administered to a living body orally or non-orally.

As shown in Figs. 1 to 3, the lighting unit 110 includes a casing 112 and an illumination member 114. The casing 112 is provided with a dark chamber 116 which blocks external light to increase contrast of an image. The dark chamber 116 serves to accommodate a living body.

Here, the casing 112 may be provided with a plurality of dark chambers 116 to accommodate a plurality of living bodies at the same time. The plurality of dark chambers 116 may be provided to a single casing 112 or may be provided to the casings 112 in one-to-one correspondence.

For convenience, in this embodiment, the lighting unit is shown as including two casings 112, and each of the casings 112 is shown as including a pair of dark chambers 116. This structure allows the dark chambers 116 to receive both hands and both feet, which are parts of a living body. It should be understood that each of the casings 112 may be formed in various shapes and formed of various materials.

Here, it is desirable that two casings 112 be supported by a frame 119 and integrally connected with each other. It should be understood that the frame 119 may be formed in various shapes and formed of various materials.

The illumination member 114 is provided at a portion of the casing 112 corresponding to the inside of the dark chamber 116 and serves to illuminate biological tissues received by the dark chamber 116.

The illumination member 114 is configured to emit excitation energy (light) at a specific wavelength to a living body (or living bodies) with a contrast agent, particularly indocyanine green (ICG) injected therein, and serves to activate ICG within a tissue of interest of the living body (or living bodies) with ICG injected therein such that fluorescent signals from the tissue of interest (biological tissue) can be observed.

Here, the excitation energy (light) emitted from the illumination member 114 has a center wavelength of 750 nm to 780 nm, which corresponds to a near-infrared region. Near-infrared rays in this wavelength range are used to observe fluorescent light through ICG administration. As the illumination member 114, a light emitting diode or laser emitting energy in the wavelength range set forth above may be used.

The illumination member 114 may include a white light illumination member which allows monitoring whether a living body is properly supported by a holding member 118 in the dark chamber 116, in addition to the near-infrared illumination member allowing the contrast agent to emit light.

Particularly, the illumination member 114 may be provided to the dark chamber 116 in a one-to-one correspondence and may be adjusted in illumination time by a controller 140. In other words, the controller 140 may control the illumination members 114 provided to the dark chambers 116 to irradiate a tissue of interest (biological tissue) with excitation energy for different periods of time. It should be understood that the controller 140 may control all of the illumination members 114 to be turned on/off at the same time.

Each of the dark chambers 116 is provided therein with a holding member 118. The holding member 118 serves to hold a corresponding part of a living body and may be deformed to allow detecting in a sitting position, a lying position, or a lying on the side position. Particularly, the holding member 118 may be formed in various shapes and formed of various materials to support a corresponding part of a living body, i.e. a hand or foot.

The inspection unit 120 serves to detect energy (fluorescent signals) in the near-infrared range emitted from a tissue of interest (or tissues of interest) *in-vivo* (contrast agent) by light irradiated to the tissue of interest *in-vivo.* Particularly, the inspection unit 120 may include a filter member 122 and a detecting member 124.

The filter member 122 is disposed in the casing 112 to correspond to each of the dark chambers 116 and serves to mainly transmit energy in the near-infrared range among energy (fluorescent signals) emitted from the tissue of interest *in-vivo* (contrast agent). Here, the range of energy in the near-infrared region allowed to pass through the filter member may vary depending upon specifications of the filter member 122 or system requirements. In other words, the filter member 122 transmits light in a specific wavelength range among fluorescent signals generated from a corresponding tissue of interest (biological tissue) due to light emitted from the illumination member 114. Specifically, the filter member 122 only transmits light having a near-infrared wavelength of 800 nm to 850 nm among fluorescent signals coming out of a living body by the illumination member 114.

As the filter member 122, a band pass filter (BPF) may be provided to each of the dark chambers 116 and can be adjusted in position and can adjust intensity of energy (fluorescent signals) having a near-infrared wavelength.

The detecting member 124 is disposed in each of the dark chambers 116 and serves to detect energy (fluorescent signals) in the near-infrared region passing through the filter member 122. Particularly, the detecting member 124 senses light having passed through the filter member 122 and converts the light into a digital signal. Specifically, the detecting member converts an analog image into digital data by changing the image into electrical signals and stores the digital data in a storage medium. For example, the detecting member 124 may be a charge-coupled device (CCD) camera among digital cameras. The detecting member 124 detects fluorescent signals that have been input to the inspection unit 120 and then passed through the filter member 122, converts the image into digital data and outputs the digital data.

The inspection unit 120 and the illumination member 114 may be disposed close to each other and the inspection unit 120 is disposed to photograph a corresponding tissue of interest (biological tissue) within the casing 112 of the lighting unit 110.

Particularly, a plurality of filter members 122 and a plurality of detecting members 124 may be provided equal in number to independently examine one or more tissues of interest and are placed within a corresponding dark chamber 116.

The plurality of filter members 122 and the plurality of detecting members 124 are connected to the controller 140 which controls operation time. The controller 140 controls operation time of all of the inspection units 120 disposed within the respective dark chambers 116. In other words, the controller 140 may control the detecting members 124 to operate for the same period of time or to operate for different periods of time.

Operation time of the detecting member 124 may be set from a time point that detecting is started to a time point that emission of near-infrared rays from the contrast agent is no longer detected, and the detecting member 124 may be controlled to perform detecting at preset time intervals. Particularly, it is desirable that the detecting member 124 be controlled to perform detecting at time intervals of several milliseconds to 10 seconds. Here, operation time of the detecting member 124 may vary depending upon the kind or dosage of the contrast agent, external temperature, and the like. The interval in which the detecting member 124 operates may be adjusted within 10 seconds.

The determination unit 130 serves to determine abnormality of perfusion and/or vessels of a tissue of interest (or tissue of interest) *in-vivo* based on the image data detected by the inspection unit 120 (resulting data for the near-infrared region).

The determination unit 130 may be further provided with an input device (not shown) for receiving the digital data from the inspection unit 120, and an output device 130 for outputting data may be connected to the determination unit 130 or the determination unit 130, as shown in Fig. 5 and Fig. 6.

Here, the digital data output from the inspection unit 120 are transmitted to the determination unit 130 through wireless or wired communication, for which RSC 232, a parallel port, IEEE 1934, or a USB is preferably used.

The determination unit 130 includes the pattern processor 132, the characteristic value calculator 134, and the diagnostic unit 136.

The pattern processor 132 serves to pattern image data obtained by detecting a tissue of interest or a region of interest (ROI) for each inspection area of a living body.

The characteristic value calculator 134 serves to calculate various characteristic values reflecting perfusion conditions (for example, blood flow conditions) from the resulting patterned data, and the diagnostic unit 136 serves to diagnose abnormality (for example, vascular disease) of vessels or perfusion of the tissue of interest *in-vivo* based on the calculated characteristic values or combinations thereof.

In other words, the pattern processor 132 processes input signals into fluorescence intensity of a tissue of interest over time in order to pattern the input signals; the characteristic value calculator 134 calculates partial characteristic values from the processed pattern of fluorescence intensity over time; and the diagnostic unit 136 diagnoses abnormality of vessels or perfusion of the tissue of interest or calculates the perfusion rate using each of the characteristic values calculated by the characteristic value calculator 134 or combinations of the characteristic values.

As such, the determination unit 130 measures partial ICG fluorescence intensity over time of ICG administered to a living body, and performs pattern analysis for each part and diagnosis of abnormality of vessels (perfusion).

The determination unit 130 may be provided to correspond to the inspection unit 120 provided to each of the dark chambers 116 in one-to-one correspondence. Alternatively, only one determination unit may be provided to be connected to a plurality of the inspection units 120. In addition, the inspection unit 120 is connected to the controller 140.

The controller 140 controls the lighting unit 110 and inspection unit 120 provided for each of different tissues of interest *in-vivo* as well as the determination unit 130, and adjusts operation time of each component of the lighting unit 110 and components of the inspection unit 120.

As used herein, the characteristic value (values) refers to a factor converted into a real number that can characterize a pattern of resulting data over time in a tissue of interest or a region of interest (ROI), and the determination unit 130 may calculate blood flow in the region of interest through comparison of the characteristic values. The characteristic value (values) may be obtained from changes over time of specific physical values (for example, ICG fluorescence intensity) in a tissue of interest, acquired from the data detected by the inspection unit 120.

When characteristic values for each tissue of interest are acquired from normal groups or abnormal groups of vessels or perfusion, a combination value of one or more characteristic values included in the normal groups or the abnormal groups allows the corresponding groups to be classified with a sensitivity and specificity of 80% or higher.

A plurality of different tissues of interest (biological tissues) are simultaneously photographed in the plurality of dark chambers 116 accommodating the illumination member 114, the filter member 122, and the detecting member 124, and the determination unit 130 compares and analyzes characteristic values calculated with respect to the plurality of tissues of interest to determine whether vessels or perfusion (blood vessels, blood flow) is normal.

Fig. 5 and Fig. 6 are views illustrating inspection of blood flow of a foot, which is one of *in-vivo* regions. In Fig. 5 and Fig. 6, unexplained reference numerals are as defined above.

The determination unit 130 determining abnormality of blood flow and blood vessels of a living body can diagnose development of vascular complication in a diabetic patient or lymphatic disease depending upon results of determination of whether blood flow and blood vessels is abnormal. In addition, the determination unit 130 can diagnose arteriosclerosis and arteriostenosis, particularly arteriosclerosis and arteriostenosis in the coronary arteries, the carotid, the femoral artery, the anterior tibial artery, and the posterior tibial artery depending upon results of determination of whether blood flow and blood vessels is abnormal. Further, the determination unit 130 can diagnose cold hands and feet, Raynaud disease, and Raynaud syndrome depending upon results of determination of whether blood flow and blood vessels is abnormal. Here, the determination unit 130 preferably represents results of determination as to whether blood flow and blood vessels are abnormal by one or more numerical values or a perfusion map.

As such, the determination unit 130 may determine occurrence of vascular complication in a diabetic patient or lymphatic diseases; may determine occurrence of arteriosclerosis and arteriostenosis, particularly, arteriosclerosis and arteriostenosis in the coronary arteries, the carotid, the femoral artery, the anterior tibial artery, and the posterior tibial artery; and may diagnose cold hands and feet, Raynaud disease, and Raynaud syndrome through the pattern processor 132, the characteristic value calculator 134, and the diagnostic unit 136.

In addition, determination or diagnosis results by the determination unit 130 are represented by one or more numerical values or a perfusion map, whereby a user or patient can easily interpret or understand the results.

In this embodiment, characteristic values, based on which abnormality of vessels or perfusion (for example, blood vessel, lymphatic vessel, blood flow, lymphatic flow, and the like) is determined, are converted into a numerical formula according to the following procedure.

In some embodiments, a graph is plotted by detecting changes in ICG fluorescence intensity in the top of a foot at 1/5 Hz for 10 minutes after in vivo injection of ICG and patterning the changes with the highest fluorescence intensity assumed as 1 (see Fig. 7).

Fig. 8 is a graph for illustrating changes in fluorescence intensity over time of a person with a normal blood flow and shows Tₘₐₓ, Plateau Tₘₐₓ, Onset, and Slope, each of which is a characteristic value.

As shown in Fig. 8, the biological tissue diagnostic apparatus 100 according to this embodiment allows blood flow of a tissue of interest (biological tissue) corresponding to hands or feet in a normal state to be represented by a change in fluorescence intensity over time.

Here, characteristic values are defined as combinations of at least one of Tₘₐₓ, Plateau Tₘₐₓ, Onset, and Slope.

Tₘₐₓ refers to a period of time to reach a point where fluorescence intensity reaches a maximum value in a patterned near-infrared emission region, and Plateau Tₘₐₓ refers to a section in which fluorescence intensity is greater than a specific value in the patterned near-infrared emission region.

Particularly, when fluorescent materials accumulate in lots of blood vessels due to poor blood flow or blood flow is delayed, a flat section in which fluorescence intensity is maintained at a high level over a certain range is observed and indicated by Plateau Tₘₐₓ.

Onset refers to a region from a time point that pattering is started (initial detection point) to a time point that a sharp inflection occurs in the curve, causing the slope of the curve to become greater than a reference slope; and Slope refers to a pattern in a downward region after Plateau Tₘₐₓ in a patterned near-infrared emission region (a period of time from a time point that fluorescence intensity starts to decrease below a reference value to a time point that fluorescence intensity is last detected).

If the Plateau section (Plateau Tₘₐₓ) in which fluorescence intensity is greater than or equal to a predetermined reference value (FI_{c}) or a section with a gradual inclination is observed, it can be said that blood flow is abnormal.

Thus, in this embodiment, after a point where fluorescence intensity is greater than or equal to a certain value is set, the period of time (Tₘₐₓ) that it takes to reach the point where ICG fluorescence intensity of ischemic tissue reaches a maximum value in the corresponding section (Plateau Tₘₐₓ) is considered to determine whether blood vessel or blood flow is abnormal. Here, as the point where fluorescence intensity is greater than or equal to a certain value, any value between 70% and 95% of the maximum fluorescence intensity may be defined.

As shown in Fig. 8, Fig. 9, and Fig. 10, when one tissue of interest (biological tissue) and another tissue of interest are simultaneously subjected to blood flow diagnosis, if difference in Tₘₐₓ value between the one tissue of interest and the other tissue of interest is greater than a first preset value, it can be determined that the blood flow is abnormal.

In addition, if a Plateau Tₘₐₓ value ratio of the other tissue of interest to the one tissue of interest is greater than a second preset value, it can be determined that the blood flow is abnormal.

Further, if an Onset value ratio of the other tissue of interest to the one tissue of interest is greater than a third preset value, it can be determined that the blood flow is abnormal.

Here, the one tissue of interest may be a toe and the other tissue of interest may be a finger. Each of the first preset value, the second preset value, and the third preset value is an average value (normal value) for normal persons.

If the blood flow is slow, this is simply represented by a pattern in which the absolute value of FI becomes smaller; Onset, Tₘₐₓ, and plateau Tₘₐₓ become longer; and Slope becomes lower. By comparing these characteristic values, blood flow of a tissue to be examined can be diagnosed and a numerical formula numerically expressing the blood flow rate can be made using the characteristic values.

In addition, characteristic values of the other tissue of interest, i.e. both hands, may be averaged or separately used. When one tissue of interest has different ICG patterns, that is, when the ICG patterns of both feet are different from each other, whether one or both legs have arteriostenosis may be diagnosed by finding characteristic values of both feet.

By way of example, there is not much difference in Tₘₐₓ and Onset between both hands and both feet in normal groups of Fig. 9, whereas, in the case of a patient with abnormal blood flow of Fig. 10, Tₘₐₓ and Onset of both hands are considerably small as compared with those of both feet and there is significant difference in Onset value between both feet.

In Fig. 10, arteriostenosis is diagnosed in both legs and it is diagnosed that the left leg has a relatively severe degree of stenosis.

Fig. 11 is a reference view illustrating a method for diagnosing an abnormal tissue of interest based on graphs obtained by patterning fluorescence intensity in a plurality of tissues of interest. As shown in Fig. 11, in trend graphs obtained by patterning fluorescence intensity in four tissues of interest, trend graphs on three tissues of interest (upper left, lower right, lower left) are synchronized in terms of shape in a certain distribution range, whereas the trend graph on the other tissue of interest (upper right), i.e. a tissue of interest of the right hand, shows a different trend therefrom. In this case, the three synchronized tissues of interest may be considered to exhibit normal blood flow or blood vessel characteristics, whereas blood flow or blood vessel conditions of the other tissue of interest may be considered abnormal. Thus, based on these characteristics, the determination unit 130 may diagnose a tissue of interest corresponding to characteristic values departing from a preset normal distribution range as an abnormal tissue of interest.

The determination unit 130 outputs diagnostic results based on the data detected by the inspection unit 120 through the output device 138. As the output device 138, a CRT monitor, an LCD, a plasma display, and the like may be used.

Fig. 4 is a flowchart illustrating a biological tissue diagnostic method according to one embodiment of the present invention. Next, a biological tissue diagnostic method according to one embodiment will be described with reference to Fig. 4. In this embodiment, one or more lighting units 110 and one or more inspection units 120 may be provided depending upon the number of tissues of interest.

First, an illumination member 114 irradiates at least one tissue of interest *in-vivo* with a contrast agent administered thereto with contrast agent excitation energy (S410), and an inspection unit 120 detects energy (energy in the near-infrared region) emitted from the tissue of interest *in-vivo* (contrast agent) (S420).

Then, a determination unit 130 determines abnormality of vessels or perfusion of the at least one tissue of interest based on image data detected by the inspection unit 120 (S430). A more detailed description of step S430 is as follows.

First, a pattern processor 132 patterns a change of the image data over time (S431). In other words, the pattern processor 132 receives electrical signals, which are detected by a detecting member 124 of the inspection unit 120 and then digitized, and process the signals, thereby patterning a trend of a specific physical value (for example, fluorescence intensity) over time.

Then, a characteristic value calculator 134 calculates at least one characteristic value based on the patterned data (S432). In other words, the characteristic value calculator 134 calculates one or more characteristic values from the patterned data or patterned graph. Here, the characteristic value (values) is obtained from a change of a specific physical value over time in a tissue of interest. The at least one characteristic value may include at least one selected from among a period of time from a time point that a physical value (for example, fluorescence intensity) is initially detected to a time point that the physical value reaches a maximum value (Tₘₐₓ); a slope with which the physical value increases or decreases for a specific period of time; a period of time for which the physical value is maintained at a reference value or more (Plateau Tₘₐₓ); a period of time from the time point that the physical value is initially detected to a time point before the physical value increases with a slope greater than or equal to a reference slope (Onset); and a period of time from a time point that the physical value starts to decrease below the reference value to a time point that the physical value is detected last (Slope).

Next, a diagnostic unit 136 diagnoses, based on the at least one characteristic value or combinations of at least two characteristic values, whether vessels or perfusion of the at least one tissue of interest *in-vivo* is abnormal. For example, in the case that one tissue of interest (biological tissue) and another tissue of interest are simultaneously subjected to blood flow diagnosis, if difference in Tₘₐₓ value between the one tissue of interest and the other tissue of interest is greater than a first preset value, it is determined that the blood flow is abnormal, and if a Plateau Tₘₐₓ value ratio of the other tissue of interest to the one tissue of interest is greater than a second preset value, it is also determined that the blood flow is abnormal. In addition, if an Onset value ratio of the other tissue of interest to the one tissue of interest is greater than a third preset value, it is determined that the blood flow is abnormal. Here, each of the first preset value, the second preset value, and the third preset value is an average value (normal value) for normal persons.

In addition, the tissue of interest may include a plurality of tissues of interest, and the determination unit 130 may diagnose an abnormal tissue of interest by comparing the characteristic values of the tissues of interest. Here, the determination unit 130 may diagnose a tissue of interest corresponding to a characteristic value departing from a preset normal distribution range as an abnormal tissue of interest. More specifically, as shown in Fig. 11, in trend graphs obtained by pattering fluorescence intensity of four tissues of interest, the trend graphs of fluorescence intensity on three tissues of interest are synchronized in shape in a certain distribution range, whereas the trend graph on the other tissue of interest shows a different trend therefrom. In this case, the three synchronized tissues of interest may be considered to exhibit normal blood flow or blood vessel characteristics, whereas blood flow or blood vessel conditions of the other tissue of interest may be considered abnormal. Thus, based on these characteristics, the determination unit 130 may diagnose a tissue of interest corresponding to a characteristic value departing from a preset normal distribution range as an abnormal tissue of interest.

As described above, the biological tissue diagnostic apparatus and method according to the present invention can continuously photograph tissues of interest *in-vivo* for a certain period of time, particularly blood vessels/lymphatic vessels which are distributed near the skin, such as peripheral tissues and carotid territories, allowing easy photographing with near-infrared light, thereby accurately diagnosing whether vessels or perfusion of the tissues of interest is abnormal.

In addition, the biological tissue diagnostic apparatus and method according to the present invention can simultaneously or selectively photograph one or more tissues *in-vivo,* specifically both hands and both feet to diagnose abnormality of blood vessels or lymphatic vessels, thereby performing accurate diagnosis throughcomplex diagnosis while reducing diagnostic time.

Although some embodiments have been described above, it should be understood that the foregoing embodiments are provided for illustration only and should not be construed in any way as limiting the scope of the present invention, and that various modifications, changes, and alterations can be made by those skilled in the art without departing from the spirit and scope of the present invention defined by the accompanying claims and equivalents thereof

## Claims

1. A biological tissue diagnostic apparatus, comprising:
at least one lighting unit irradiating at least one tissue of interest *in-vivo* with a contrast agent administered thereto with contrast agent excitation energy;
at least one inspection unit detecting energy emitted from the at least one tissue of interest; and
a determination unit determining abnormality of vessels or perfusion of the at least one tissue of interest based on image data detected by the at least one inspection unit.

2. The biological tissue diagnostic apparatus according to claim 1, wherein the determination unit comprises:
a pattern processor patterning a change of the image data over time;
a characteristic value calculator calculating at least one characteristic value based on patterned data; and
a diagnostic unit diagnosing abnormality of vessels or perfusion of the at least one tissue of interest based on the at least one characteristic value or combinations thereof

3. The biological tissue diagnostic apparatus according to claim 2, wherein the tissue of interest *in-vivo* comprises a plurality of tissues of interest, and the diagnostic unit further diagnoses an abnormal tissue of interest through comparison of characteristic values of the tissues of interest.

4. The biological tissue diagnostic apparatus according to claim 3, wherein, in diagnosis of an abnormal tissue of interest, the determination unit diagnoses a tissue of interest corresponding to a characteristic value departing from a preset normal distribution range of the characteristic values of the tissues of interest as an abnormal tissue of interest.

5. The biological tissue diagnostic apparatus according to claim 3, wherein when characteristic values for each tissue of interest are acquired from normal groups or abnormal groups of vessels or perfusion, a combination value of one or more characteristic values included in the normal groups or the abnormal groups allows the corresponding groups to be classified with a sensitivity and specificity of 80% or higher.

6. The biological tissue diagnostic apparatus according to claim 2, wherein the at least one characteristic value is obtained from a change of a specific physical value for a first time period in the tissue of interest obtained from the image data, and
wherein the at least one characteristic value obtained for the first time period comprises at least one selected from among a period of time from a time point that the physical value is initially detected to a time point that the physical value reaches a maximum value; a slope with which the physical value increases or decreases for a specific period of time; a period of time for which the physical value is maintained at a reference value or more; a period of time from the time point that the physical value is initially detected to a time point before the physical value increases with a slope greater than or equal to a reference slope; and a period of time from a time point that the physical value starts to decrease below the reference value to a time point that the physical value is last detected.

7. The biological tissue diagnostic apparatus according to claim 1, wherein the contrast agent is an indocyanine green (ICG) pigment.

8. The biological tissue diagnostic apparatus according to claim 1, wherein the inspection unit comprises:
a filter member transmitting energy in the near-infrared region among energy emitted from the contrast agent; and
a detecting member detecting energy in the near-infrared region having passed through the filter member.

9. The biological tissue diagnostic apparatus according to claim 8, wherein the biological tissue diagnostic apparatus comprises a plurality of the inspection units to independently inspect one or more biological tissues, and
the detecting member included in each of the inspection units is controlled in operation time by a controller.

10. The biological tissue diagnostic apparatus according to claim 8, wherein operation time of the detecting member is within a period of time from a time point that detecting is started to a time point that emission of near-infrared rays from the contrast agent is no longer detected, and
the detecting member is controlled to perform detecting at preset time intervals.

11. The biological tissue diagnostic apparatus according to claim 1, wherein the determination unit determining abnormality of vessels or perfusion of the at least one tissue of interest represents determination results as to abnormality of the vessels or perfusion by one or more numerical values or a perfusion map.

12. The biological tissue diagnostic apparatus according to claim 1, further comprising:
a dark chamber blocking external light to increase contrast of an image,
wherein the lighting unit comprises an illumination member emitting the contrast agent excitation energy, and the dark chamber accommodates the illumination member and the inspection unit.

13. A biological tissue diagnostic method, comprising:
irradiating, by at least one lighting unit, at least one tissue of interest *in-vivo* with a contrast agent administered thereto with contrast agent excitation energy;
detecting, by at least one inspection unit, energy emitted from the at least one tissue of interest; and
determining, by a determination unit, abnormality of vessels or perfusion of the at least one tissue of interest based on image data detected by the at least one inspection unit.

14. The biological tissue diagnostic method according to claim 13, wherein determining abnormality of vessels or perfusion of the at least one tissue of interest comprises:
patterning a change of the image data over time;
calculating at least one characteristic value based on the patterned data; and
diagnosing abnormality of vessels or perfusion of the at least one tissue of interest based on the at least one characteristic value or combinations of the characteristic values.

15. The biological tissue diagnostic method according to claim 14, wherein the tissue of interest *in-vivo* comprises a plurality of tissues of interest, and
wherein determining abnormality of vessels or perfusion of the at least one tissue of interest further comprises:
diagnosing an abnormal tissue of interest through comparison of the characteristic values of the tissues of interest.

16. The biological tissue diagnostic method according to claim 15, wherein, in diagnosing an abnormal tissue of interest, the determination unit diagnoses a tissue of interest corresponding to characteristic values departing from a preset normal distribution range as an abnormal tissue of interest.

17. The biological tissue diagnostic method according to claim 14, wherein the at least one characteristic value is obtained from a change of a specific physical value for a first time period in the tissue of interest obtained from the image data, and
wherein the at least one characteristic value obtained for the first time period comprises at least one selected from among a period of time from a time point that the physical value is initially detected to a time point that the physical value reaches a maximum value; a period of time for which the physical value is maintained at a reference value or more; a period of time from the time point that the physical value is initially detected to a time point before the physical value increases with a slope greater than or equal to a reference slope; and a period of time from a time point that the physical value starts to decrease below the reference value to a time point that the physical value is last detected.
